# EUROPEAN PATENT SPECIFICATION

(11) **EP 1 480 583 B1**
(45) Date of publication and mention of the grant of the patent: **19.11.2014**
(21) Application number: 02793687.1
(22) Date of filing: 19.12.2002
(51) Int. Cl.: A61F 2/30, A61F 2/44, A61F 2/46

(54) **METHOD FOR MANUFACTURING A HUMAN INTERVERTEBRAL IMPLANT**
VERFAHREN ZUR HERSTEUNG EINES INTERVERTEBRALEN IMPLANTAT FÜR MENSCHEN
PROCEDE DE PRODUCTION D'UN IMPLANT INTERVERTEBRAL HUMAIN

(30) Priority: 20.12.2001 SE 0104323
(43) Date of publication of application: 01.12.2004
(73) Proprietor: Ortoma AB, 411 26 Gothenburg (SE)
(72) Inventor: ANDERSSON, Matts, S-443 39 Lerum (SE); HOLM, Sten, S-435 41 Mölnlycke (SE)
(74) Representative: Byström, Kurt Linus
(86) International application number: PCT/SE2002/002383
(87) International publication number: WO 2003/059211

(56) References cited:
- WO-A1-01/85040
- DE-A1- 3 522 196
- US-A- 5 370 692
- US-A- 5 545 229
- US-A- 5 762 125
- US-A- 5 946 370
- US-A- 6 112 109
- US-A1- 2001 004 710

## Description

The present invention refers i.a. to a method for producing biocompatible Implants for a human intervertebral disk or a vertebral disk in the human body, where the present condition (or state) is established by means of condition establishing (state establishing) means or equipment, e.g. computer tomography, and parameters, e.g. age, weight, flexibility, etcetera.

In connection to treatment of different types of vertebral disk problems it has been started out from the standpoint that the patient is not to be treated until apparent inconvenience has occurred. The symtoms of the inconvenience can manifest themselves in different manners as pains, stiffness, limited agility, etcetera. It has thereby been born in mind the situation of back trouble which has progressed and in many cases proposed drastic measures, e.g. arthrodesis, which have caused the patient larger or smaller permanent disabilities.

A very large part of the population (70-80%) will experience back pains during their lifetime. These pains often give raise to sick-listing and social/economic problems. The origin of back pain in many cases can be referred to the function of the intervertebral disk. At injury/wear or degeneration (In many cases age alterations and in some cases genetics) the intervertebral disk is so altered that its mechanical properties are impaired and the nerves can be squeezed.

There are apparent wishes for improved treatment methods, which can reduce the risk for permanent and advanced disabilities in connection to back problems. The present invention Intends to solve i.a. this set of problems and it is based on the understanding that back problems can be forseen at an early stage before the patient have had time to develop a more wide-spread clinical picture. In accordance with the basic idea of the invention it shall be possible to take measures at very early pre-stages of back pain for a predestinated patient.

The requirement for adequate surgical techniques and appropriate replacements for degenerated intervertebral disks is therefore important. Problem of technically arranging for production of appropriate implants and to arrange efficient methods and arrangements for production of the implant thereby occur. The Invention intends to solve this problem. Hereby it is desired that it is possible to use technique which is well-tested and known per se, and which can be transferred to this special area. The technical arrangement shall be able to operate with safe examination and analysis methods as a starting point. The invention also solves this set of problems.

US 2001/004710 A discloses a method for producing a biocompatible implant i.a. for a vertebral disk, wherein a holographic image can be generated through an arthroscope, and where a computer program can be used in order to assist the operator in producing a component in order to provide optimal alignment of the joint involved. The method incorporates use of an implantable mould apparatus comprising a cavity adapted to receive and contain a flowable biomaterial.

The invention is defined in claim 1

In accordance with the basic idea of the present invention it is intended, at earlier degenerative alterations to implant a substitute for the entire or a part of the centre portion of the intervertebral disk of the lumbar portion. Specific diagnostics and evaluation of MR/CT pictures give dimensions specific for the patient for producing the implant(-s).

The features that primarily can be said to be characterzing for a method according to the invention is that the state of the disk is established by means of computer tomography and parameters representing age, weight, flexibility etc, and that said second information Is created by the computer equipment in dependency of the simulation and Incorporates information about the outer volume, elasticity, resiliency, choice of material and design of the desired implant.

A favourable embodiment of the new method is defined in subclaims thereof.

By means of what has been suggested hereabove it is possible that required operations can be simplified and be made less comprehensive as compared to earlier. The implant in question can be made thus that the progress of the course of illness is prevented or counteracted to a substantial degree. Comparatively early substitution of a functional implant in an injured back segment is very important for the possibilities of the patient for a good and rapid rehabilitation from strong backpains. Conservative spinal surgery and a motion promoting implant have an economical potential as well as a considerable sociomedical importance.

The method and arrangement proposed at present shall be described hereinafter with reference to the accompanying drawings, wherein
- Figure 1: in the form of a block diagram shows analysis of a disk function in question, Initiation of information to the computer equipment, simulation function in the computer equipment, and transfer of control information to a production unit via a communication system,
- figures 2-2a: show in a vertical section a normal disk and vertebra function in a human body,
- figures 3-3a: show in longitudinal section and in cross section, respectively a disk function with Annular Degeneration,
- figures 4-4a: show in longitudinal section and in cross section, respectively a disk function with Nuclear Herniation,
- figure 5: shows in cross section the design of an Implant in the case according to figures 3-3a,
- figure 6: shows in cross section the design of an implant in the case according to figure 4a,
- figure 7: shows in vertical section a first embodiment of an implant,
- figure 8: shows In vertical section a second embodiment of an Implant,
- figure 9: shows in vertical section and In principle a third embodiment of an implant,
- figure 10: shows in vertical section and in principle a fourth embodiment of an implant,
- figure 11: shows in a horizontal view and in principle the embodiment according to figure 10, and
- figure 12: shows schematically the arrangements for identification and production of different types of implants.

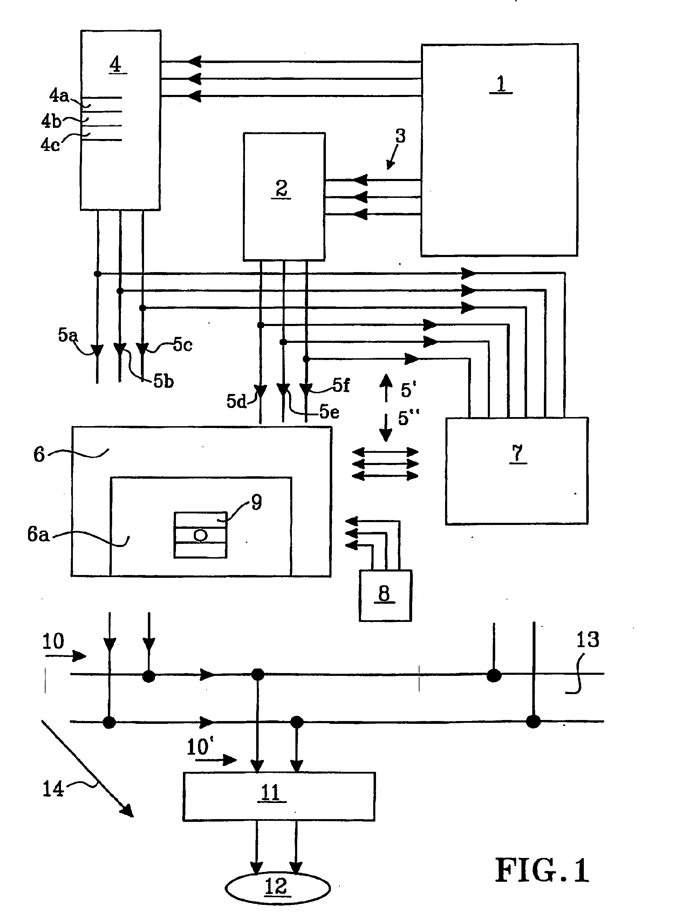

In figure 1 a not shown vertebral disk situation is designated 1. The vertebral disk situation can be mapped in a manner known per se, by means of computer tomography, which is symbolized by 2 In figure 1. Information collected from the situation according to 1 to the equipment 2 is indicated by arrows 3. Furthermore the figure incorporates a block 4, which represents information about symbolically indicated parameters 4a, 4b, 4c, which can represent age, weight and flexibility, respectively. In block 4 the parameters in question are thus taken in from the current situation in accordance with block 1. Further examination methods 2 and parameters can be at hand. By aid of the said means and parameters is created a first Information 5, which In the figure is composed by the informations 5a, 5b, 5c, 5d, 5e and 5f. The first information Is inputted In a computer equipment 6 which can be of a type known per se. The present means and parameter functions can be transferred to memory devices 7, see arrow 5'. The said first information can be stored temporarily or for long-term in the memory devices 7. The first Information 5 thus can be transferred directly to the computer device 6 or via the memory devices 7, from where the first Information is transferred to the computer equipment 6, see arrow 5", or to another computer if such is used. On the computer screen in the computer equipment 6 is generated a simulation of the vertebral disk situation present in the block 1. In the simulated model additional information can be Introduced In a manner known per se. By means of the additional Information it Is possible to create a visual picture of how the implant for the situation at hand shall look in the optimum case. In connection to this creation it is possible to use knowledge based on experience and which e.g. can be collected from the block 4 or from a library 8. The desired solution aimed at, of an Implant for vertebra and disk arrangements 9 in question, thus can be obtained and from the computer equipment can be emitted a second information 9, which represents the design and composition, e.g. outer volume or design of the implant desired or aimed at. The second Information is utilized as manufacturing information, which can be transferred via tele and/or computer systems, e.g. via the official telephone network, Internet, etcetera, to a manufacturing unit 11, which can be located in connection to the examination equipment and/or the simulation equipment as described above. Alternatively, the manufacturing equipment can be given a central location, which serves a number of examining and/or simulating functions. The manufacturing equipment receives the second information 10, 10' on which is based the manufacture of a desired or aimed at implant 12 as described above and produced in the manufacturing equipment. The second information Initiates or participates in control functions for machine or machines provided In the equipment 11. Also this part can be effected in a manner known per se and shall here not be described more in detail. The transfer in the case illustrated is designated 13 and the memory means 7 can certainly be connected to this or those information transferring signals and e.g. deliver the computer equipment 6 with its information (compare the arrows 5"). The second information can also be transferred directly by mail or other delivery, which is indicated with the arrow 14.

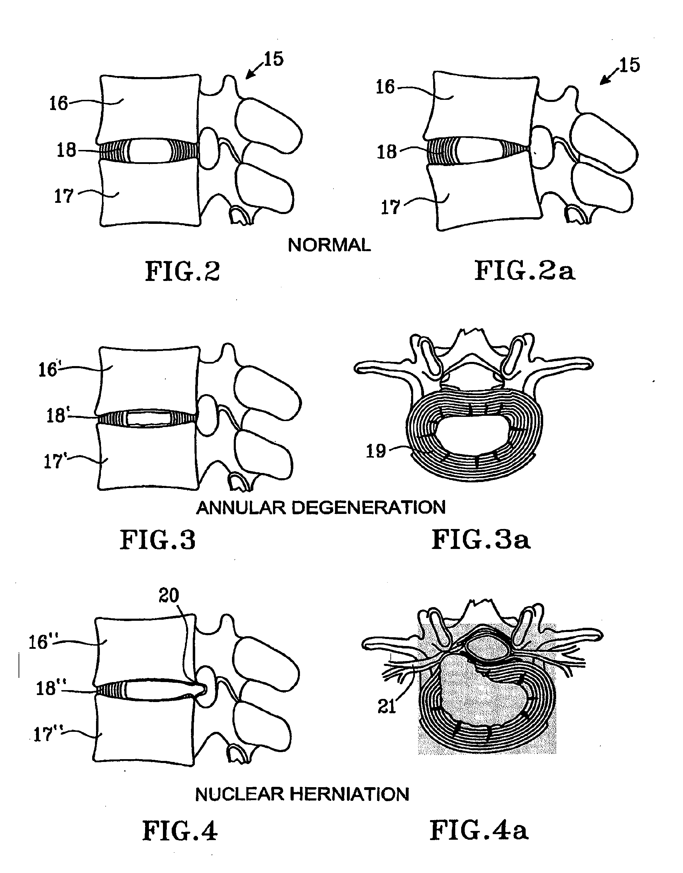

A spinal column in the human body is designated 15 In Fig. 2 and two vertebras positioned adjacent each other with 16 and 17. An intervertebral disk or a vertebral disk is designated 18. Fig. 2 thereby shows an initial position for the vertebras and the disk/lamina, whereas Fig. 2a shows a bent spinal column, where the vertebras have been put at angles to each other and the disk has adapted itself to the angled position. Figs. 2 and 2a show a normal case with uninjured vertebras 16 and 17 and disk 18. In the case according to Figs. 3 and 3a there Is a degeneration of the lamina or disk 18' between the vertebras 16' and 17'. The disk 18' has been compressed and deformed and the flexibility of the spinal column has been Impaired. The inner part of the disk or the lamina has cracks and such a crack which extends from the inner part and outwards has been designated 19. Figs. 3 and 3a represent "Annular Degeneration".

In the case according to Figs. 4 and 4a there is a matter of "Nuclear Hernitation". In this case there is a protuberance 20 on the membrane enclosing the disk and the protuberance presses on a nerve 21, which results In that the patient may suffer from pains.

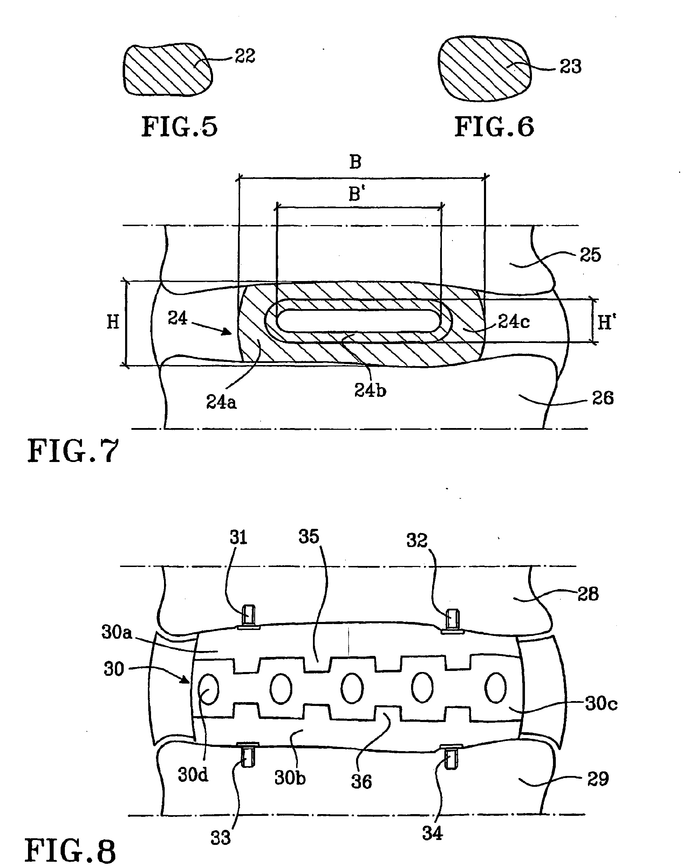

Figs. 5 and 6 intend to show the outer volume or design of implants 22, 23, which can be adapted to the relevant internally formed spaces in the different disks. By means of microsurgery it is possible to fill out the spaces and further degeneration with cracks and protuberances are prevented.

The above mentioned first and second informations can be based on digital transfer between the different components. Regarding the different vertebra and disk situations it is referred to the well-known litterature.

In Fig. 7 is shown an implant 24 for a complete intervertebral disk, which has been completely substituted by the implant. The vertebras have the reference numbers 25 and 26. The implant can be formed In a plastic material, e.g. polyurethane, with varying Shore-number. Thus an outer part 24a can for instance have a first Shore-number (Shore-modulus) and one or more inner parts 24b another Shore-number (Shore-modulus). The first Shore-number may be bigger than the second Shore-number and vice versa depending on vertebra and disk situation in question. The implant has an overall height H of 12 ± 5 mm and an overall width B of 20 ± 5 mm and a length (perpedicular to the plane of the drawing) of 40 ± 5 mm. The corresponding measures H', B' and length for the inner part 24b are below the first mentioned measures In dependency of the wall thickness 24c, which shall be obtained and they are for instance 5 - 10 mm smaller. The Shore-numbers can be chosen between 20-100.

In Fig. 8 the surrounding vertebras are designated 28 and 29. In this case the implant 30 is formed by a laminate with outer parts, e.g. outer disks 30a and 30b made from titanium and/or ceramics. The outer disks are attached to the vertebras by means of fixing members 31, 32, 33 and 34, e.g. In form of fixing screws. Between the outer disks is provided an intermediate layer 30c made from polyurethane (plastics). The intermediate layer is attached to the outer parts by means of interconnection arrangements, e.g. in the form of undercut connections 35 and 36. In one embodiment the Intermediate part may be provided with recesses 30d by means of which elasticity, flexibility, etcetera may be optimized. The intermediate part can be made with the same Shore-number or with different Shore-numbers in its different parts and the Shore-number(-s) can be chosen between 20-100.

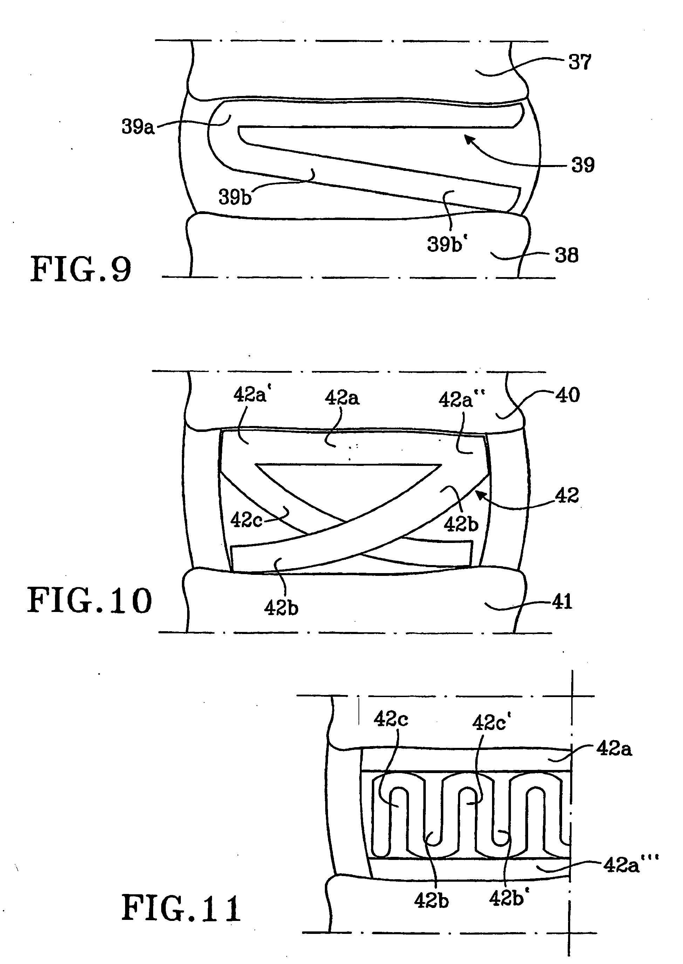

In the embodiment according to Fig. 9 the vertebras are designated 37 and 38 and an implant provided with a spring or springs with 39. The implant Incorporates a part 39a attached to or attachable to the vertebra 37 and one or more resilient part(-s) 39b attached thereto and which engage or is/are attached to an inner surface of the vertebra 38 via its or their ends 39b'. The parts 39a, 39b, 39b' can be made from titanium. A conventional not shown spring can also be fitted between the parts. The spring(-s) can be curved in the vertical section.

In Fig. 10 the vertebras are designated 40 and 41 and the implant 42. A connecting part 42a is arranged to be connectable to the vertebra 40 and In the ends 42a' and 42a" of the part 42a are fitted resilient members 42b and 42c, which engage or are attached to the vertebra 41. The parts 42a, 42b and 42c can be made from titanium and/or ceramics.

Fig. 11 shows an arrangement with a plurality of springs 42b, 42b', etcetera and 42c, 42c', etcetera. It is possible that there are alternatively two parts 42a, 42a" If a double arrangement is wanted.

Fig. 12 intends to show an example of i.a. a tangible reading situation on a human intervertebral disk. The reading can be based on establishment of current degeneration, which In the figure has been shown with a degeneration/age curve 43 (e.g. male sex) and 43' (female sex). Contribution/age curves corresponding to the degeneration are shown at 44, 44'. The reading of the curves 43, 43' can be complied In a unit 45 and the evaluation can be carried through with analysis instruments/user 46. A simulation function may be set up by aid of a simulation unit 47 (compare above) and an analysis instrument 48 connetected thereto. The contribution function need not be put in relation to the degeneration function, but can be related to the wanted optimum case, compare the implant design according to Fig. 7. The reading and simulation functions can be included in a common unit function or can be divided in two different functions 49 and 50. One or both of these functions can be effected remotely over the communication network acording to the above, and which is here designated 51, 52, 53 and 54, which communication parts can be uni-directional or bi-directional from a communication point of view, see arrows. The arrangement can be provided with ordering and/or payment arrangements 55, which can be of a type known per se and which can be established, e.g. via communication parts 56, 57, 58. The reading and simulation functions can be connected to a central function 59 for simulation 60, controlling of mechanical equipment 61, economy function and design function. The arrangement can also include a test function 64 and a manufacturing function 65. The unit 59 in accordance with the above is connected or manned with user function 66, library function 67, etcetera. The central equipment 59 is arranged as a system part 68, but it may entirely or partly be closer connected to said reading and simulation functions. The simulation function can be connected to or use a testing unit 69 via a connection 70, which can be uni-directional or bi-directional. An implant 71, 71', 71" and 71'" can be subjected to tests, e.g. compression and expansion tests and flexural tests in equipment 72, 72a. By means of communications and controls 73, 74, 75 and 76 the simulation function can be established or accepted by way of a model. Also the manufacturing unit can be connected to or utilized for testing produced implants or partial functions in these. The equipment 59, 60 operates with a comparison function, in which simulation and reading can be compared and control signals to the manufacturing unit 65 can be transferred via connections 76, 77, 78, 79, 80, 81, 82, 83, 84. The control thereby is introduced to units 85, 86, 87, 88, 89, 90 and 91 depending on if the implant production shall be based on ceramics, titanium, plastics, laminates, etcetera, and on current elasticity, resilience, size, etcetera or combinations thereof. Said units 85-91 are feed-back connected to or In the system via the connection 92 and the feed-back unit 83, via which results and requests relating to the manufacture can be fed back to the central unit 59, the testing equipment 64, the reading and simulation equipments 49, 50, the library, the user, etcetera. By the invention is obtained an efficient system for sensing, simulation, testing, manufacture, ordering, etcetera, where the manufacturing result etcetera can be given a clear function and be used for the later use of the entire or parts of the system.

The invention is not limited to the embodiment shown in the above as an example but can be subjected to modifications within the scope of the accompanying claims.

## Claims

1. A method for producing a biocompatible implant (12) for a vertebral disk, preferably an intervertebral disk, in the human body, comprising the steps of:
a) establishing the present state of the disk by condition establishing means (2) and parameters,
b) creating by said condition establishing means (2) and parameters a first information (5) which is transferred to a computer equipment (6),
c) controlling with the first information the computer equipment to produce a simulation model of the present structure of the vertebral disk in question and its surrounding vertebras,
d) putting the outer volume of the implant in relation to the simulation model of the present structure of the disk,
e) creating by the computer equipment a second information (9) related to the outer volume and design of the implant in dependency of the simulation model, and
f) transferring said information (9) to one or more manufacturing equipments (11) for production of the implant.

2. The method as claimed in claim 1, **characterized in that** details about condition and/or design is transferred to one or more equipments (45), that basic details for the simulation are supplied to one or more second equipments (47), that information related to the reading and simulation are compared in one or more third equipments (59), that fourth equipments (85-91) are supplied with production information based on the reading, simulation and comparison functions, and that the simulations and the implants (12) manufactured are preferably tested in a testing equipment (64).

3. The method as claimed in claim 1, **characterized in that** the second information (9) incorporates information about such choice of material as plastics, ceramics, titanium and/or laminate composition of the implant (12).

4. The method as claimed in claim 2, **characterized in that** one or more of the first equipments (45) are arranged to effect or participate in establishment of the condition and/or the design, that one or more of the second equipments (47) are arranged to effect simulation for the present situation, that one or more of the third equipments are arranged to make comparisons of the reading and simulation and transfer in dependency of the comparison manufacturing information to one or more manufacturing equipments (11).

5. The method as claimed in claim 2, **characterized in that** models of the simulated and/or manufactured implants (12) are tested in a testing equipment (64).

6. The method as claimed in claim 1, comprising establishing the present state of the disk by means of computer tomography.

7. The method as claimed in claim 1, comprising establishing the present state of the disk by means of parameters comprising age, weight, flexibility, etcetera.

8. The method as claimed in claim 1, comprising incorporating in the second information, information about elasticity, resiliency, and/or laminate composition of the implant.

## Patentansprüche

1. Verfahren zum Herstellen eines biologisch verträglichen Implantats (12) für eine vertebrale Scheibe, vorzugsweise eine Bandscheibe, im menschlichen Körper, das die folgenden Schritte umfasst:
a) Feststellen des derzeitigen Zustands der Scheibe durch Zustandsfeststellungsmittel (2) und Parameter,
b) Erzeugen von ersten Informationen (5) durch die 2ustandsfeststellungsmittel (2) und die Parameter, die an eine Computerausrüstung (6) übertragen werden,
c) Steuern der Computerausrüstung mit den ersten Informationen, um ein Simulationsmodell der derzeitigen Struktur der betreffenden vertebralen Scheibe und ihrer umgebenden Wirbelknochen zu erzeugen,
d) Setzen des äußeren Volumens des Implantats in Beziehung zu dem Simulationsmodell der derzeitigen Struktur der Scheibe,
e) Erzeugen von zweiten Informationen (9) bezüglich des äußeren Volumens und des Designs des Implantats in Abhängigkeit von dem Simulationsmodell durch die Computerausrüstung und
f) Übertragen der Informationen (9) an eine oder mehrere Herstellungsausrüstungen (11) für die Herstellung des Implantats.

2. Verfahren nach Anspruch 1, **dadurch gekennzeichnet, dass** Details über den Zustand und/oder das Design an eine oder mehrere Ausrüstungen (45) übertragen werden, dass wesentliche Details für die Simulation an eine oder mehrere zweite Ausrüstungen (47) übertragen werden, dass Informationen bezüglich des Lesens und der Simulation in einer oder mehreren dritten Ausrüstungen (59) verglichen werden, dass vierte Ausrüstungen (85-91) mit Produktionsinformationen aufgrund der Lese-, Simulations- und Vergleichsfunktionen beliefert werden und dass die Simulationen und die hergestellten Implantate (12) vorzugsweise in einer Testausrüstung (64) getestet werden.

3. Verfahren nach Anspruch 1, **dadurch gekennzeichnet, dass** die zweiten Informationen (9) Informationen über solche Wahlen des Materials wie Kunststoff, Keramik, Titan und/oder Laminatzusammensetzungen des Implantats (12) enthalten.

4. Verfahren nach Anspruch 2, **dadurch gekennzeichnet, dass** eine oder mehrere der ersten Ausrüstungen (45) ausgelegt sind, ein Feststellen des Zustands und/oder das Design auszuführen oder dazu beizutragen, dass eine oder mehrere der zweiten Ausrüstungen (47) ausgelegt sind, die Simulation für die derzeitige Situation auszuführen, dass eine oder mehrere der dritten Ausrüstungen ausgelegt sind, Vergleiche des Lesens und der Simulation auszuführen und Herstellungsinformationen in Abhängigkeit von dem Vergleich an eine oder mehrere Herstellungsausrüstungen (11) zu übertragen.

5. Verfahren nach Anspruch 2, **dadurch gekennzeichnet, dass** Modelle der simulierten und/oder hergestellten Implantate (12) in einer Testausrüstung (64) getestet werden.

6. Verfahren nach Anspruch 1, das umfasst, den derzeitigen Zustand der Scheibe mit Hilfe von Computertomographie festzustellen.

7. Verfahren nach Anspruch 1, das umfasst, den derzeitigen Zustand der Scheibe mit Hilfe von Parametern, die Alter, Gewicht, Flexibilität und dergleichen umfassen, festzustellen.

8. Verfahren nach Anspruch 1, das umfasst, Informationen über Elastizität, Rückfederung und/oder Laminatzusammensetzung des Implantats in die zweiten Informationen aufzunehmen.

## Revendications

1. Procédé pour produire un implant biocompatible (12) pour un disque vertébral, de préférence un disque intervertébral, dans le corps humain, comprenant les étapes suivantes :
a) établir l'état actuel du disque par des moyens et paramètres d'établissement de conditions (2),
b) créer, par lesdits moyens et paramètres d'établissement de conditions (2) une première information (5) qui est transférée vers un équipement informatique (6),
c) contrôler, avec la première information, l'équipement informatique pour produire un modèle de simulation de l'actuelle structure du disque vertébral en question et de ses vertèbres adjacentes,
d) mettre le volume extérieur de l'implant en relation avec le modèle de simulation de l'actuelle structure du disque,
e) créer, par l'équipement informatique, une seconde information (9) liée au volume extérieur et au dessin de l'implant en fonction du modèle de simulation, et
f) transférer ladite information (9) à un ou plusieurs équipements de fabrication (11) pour une production de l'implant.

2. Procédé tel que revendiqué dans la revendication 1, **caractérisé en ce que** des détails sur une condition et/ou un dessin sont transférés vers un ou plusieurs équipements (45), que des détails de base pour la simulation sont fournis à un ou plusieurs deuxièmes équipements (47), que des informations liées à la lecture et la simulation sont comparées dans un ou plusieurs troisièmes équipements (59), que des quatrièmes équipements (85-91) sont fournis avec des informations de production sur la base des fonctions de lecture, de simulation et de comparaison, et que les simulations et les implants (12) fabriqués sont de préférence testés dans un équipement de test (64).

3. Procédé tel que revendiqué dans la revendication 1, **caractérisé en ce que** la seconde information (9) incorpore une information sur tel choix de matériau comme une composition à base de plastique, de céramique, de titane et/ou stratifiée de l'implant (12).

4. Procédé tel que revendiqué dans la revendication 2, **caractérisé en ce que** un ou plusieurs des premiers équipements (45) sont conçus pour effectuer ou participer à l'établissement de la condition et/ou du dessin, **en ce que** un ou plusieurs des deuxièmes équipements (47) sont conçus pour effectuer une simulation pour la présente situation, **en ce que** un ou plusieurs des troisièmes équipements sont conçus pour établir des comparaisons de la lecture et de la simulation et transférer, en fonction de la comparaison, des informations de fabrication à un ou plusieurs équipements de fabrication (11).

5. Procédé tel que revendiqué dans la revendication 2, **caractérisé en ce que** des modèles des implants simulés et/ou fabriqués (12) sont testés dans un équipement de test (64).

6. Procédé tel que revendiqué dans la revendication 1, comprenant d'établir l'état actuel du disque au moyen d'une tomodensitométrie.

7. Procédé tel que revendiqué dans la revendication 1, comprenant d'établir l'actuel état du disque au moyen de paramètres comprenant l'âge, le poids, la flexibilité, etcetera.

8. Procédé tel que revendiqué dans la revendication 1, comprenant d'incorporer dans la seconde information, une information sur l'élasticité, la résilience, et/ou la composition stratifiée de l'implant.
